# EUROPEAN PATENT SPECIFICATION

(11) **EP 3 624 869 B1**
(45) Date of publication and mention of the grant of the patent: **30.06.2021**
(21) Application number: 18730855.6
(22) Date of filing: 16.05.2018
(51) Int. Cl.: A61M 1/16, A61M 1/36

(54) **FILTERING SYSTEM FOR CONTINUOUS RENAL REPLACEMENT THERAPIES**
FILTERSYSTEM FÜR KONTINUIERLICHE NIERENERSATZTHERAPIEN
SYSTÈME DE FILTRATION POUR THÉRAPIES DE REMPLACEMENT RÉNAL EN CONTINU

(30) Priority: 16.05.2017 IT 201700052603
(43) Date of publication of application: 25.03.2020
(73) Proprietor: Medica S.p.A., 41036 Medolla (IT)
(72) Inventor: BAGNOLI, Davide, 41036 Medolla (MO) (IT); CARONNA, Marco, 20133 Milano (IT); MEZZETTI, Luca, 41013 Castelfranco Emilia (MO) (IT)
(74) Representative: Cataldi, Giulia
(86) International application number: PCT/IB2018/053432
(87) International publication number: WO 2018/211434

(56) References cited:
- EP-A1- 2 679 257
- EP-A2- 1 424 089
- WO-A1-2011/128748
- CN-A- 105 688 301
- CN-U- 202 270 211
- US-A- 6 117 100

## Description

### PRIORITY CLAIM

This application claims priority from Italian Patent Application No. 102017000052603 filed on May 16, 2017.

### TECHNICAL FIELD

The present patent application relates to a filtering system for continuous renal replacement therapies.

### BACKGROUND ART

It is known that in Continuous Renal Replacement Therapies (CRRT), it may become necessary to change the dialyser filter. There may be many reasons for this: the main cause is poor anticoagulation, for example due to specific clinical conditions of the patient. Another cause can be a breakage of the filter fibres.

Finally, there are adsorbent filters, i.e., filters that retain specific molecules, that have a much shorter duration than the potential duration of whole set of tubes.

Currently, in the case of filter change, the sequence of operations is lengthy and requires the entire extracorporeal circuit to be changed, as the blood must first be returned, then the entire circuit must be replaced, and finally a new line filling step, generally known as priming, must be performed before reconnecting the patient. All this can last up to thirty minutes, during which the patient receives no treatment.

There are systems that, utilising branches in the tubing and manual clamps, make it possible to switch to a new filter, prepared in advance during the priming step. However, in these systems there is no guarantee that complete return of the blood of the first filter has taken place and, moreover, switching must be implemented manually, with all the risks of error that this entails.

Another drawback in CRRT treatments, in particular those combined with CO2 removal, is the need to achieve high flow rates and this is complicated by the risk of haemolysis due to the use of peristaltic pumps and by the limit of the sections of the tubes, which cannot be too large.

A further drawback is that it is not possible to perform two treatments in parallel, which would instead be necessary when wishing to combine treatments with filters that have a very different duration and/or very different blood flows.

### DISCLOSURE OF INVENTION

The object of the present invention is to provide a filtering system for continuous renal replacement therapies, which makes it possible to eliminate the aforesaid drawbacks.

According to the present invention there is provided a filtering system for continuous renal replacement therapies comprising the technical features as defined in claim 1. The filtering system allows automatic filter change. Preferred technical features are defined in the dependent claims.

### BRIEF DESCRIPTION OF THE DRAWINGS

The invention will now be described with reference to the accompanying drawings, which illustrate non-limiting examples thereof, wherein:
- Fig. 1 is a schematic view of a first embodiment of the filtering system not forming part of the present invention as claimed;
- Fig. 2 is a schematic view of a second embodiment of the filtering system not forming part of the present invention as claimed;
- Fig. 3 is a schematic view of a third embodiment of the filtering system according to the present invention.

### BEST MODE FOR CARRYING OUT THE INVENTION

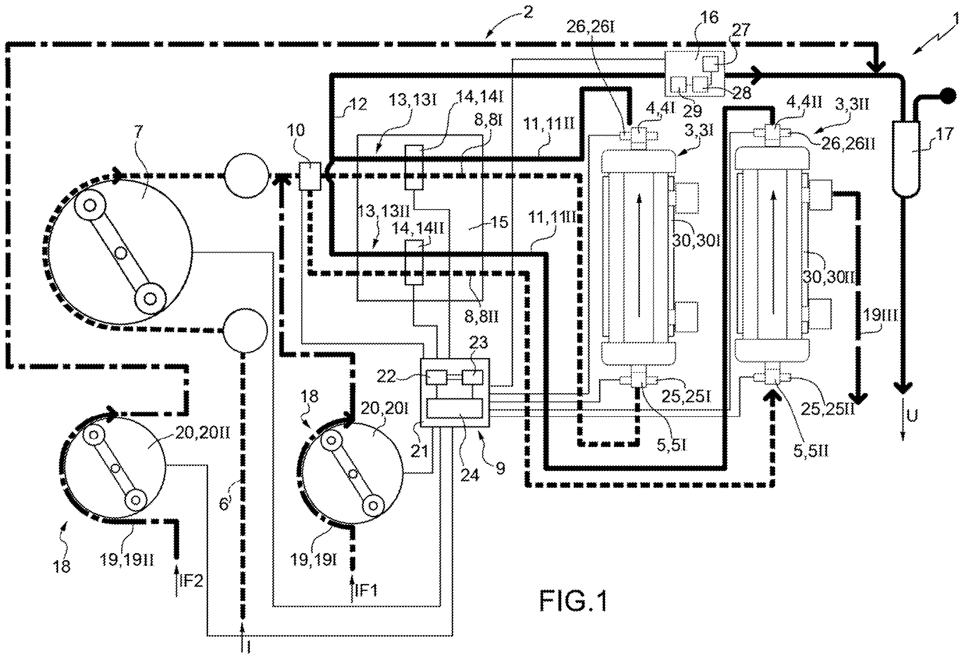

In Fig. 1, the numeral 1 indicates as a whole a filtering system 1. The filtering system 1 has an inlet I for the inflow of blood to be treated and an outlet U for the outflow of treated blood. The inlet I and the outlet U are connected to each other through an extracorporeal blood circuit 2. The exchange of blood takes place directly with the patient and the inflow and outflow of blood take place in a known manner.

Advantageously, the blood circuit 2 comprises a plurality of treatment seats 30 installed in parallel to one another and interposed between the inlet I and the outlet U. Each seat 30 is configured to accommodate a respective dialyser filter 3. Each filter 3 can be mounted releasably inside the respective seat. Optionally, each filter 3 can be replaced by a by-pass element (of known type and not illustrated).

According to the example illustrated, the blood circuit 2 comprises two seats 30 with two respective dialyser filters 3 installed in parallel.

It must be noted that in the figures the characters I and II are used to identify the shared components of a same filter 3. Each filter 3 comprises, in a known manner, an inlet mouth 4 and an outlet mouth 5. The filters 31 and 311 are filters of known type and are removable and replaceable. For example, after a given use the filters 31 and 311 must be replaced with new filters 31 and 311.

The blood circuit 2 further comprises a delivery duct 6 of the blood to be treated that connects the inlet I to a blood pump 7, in particular to a peristaltic pump of known type and illustrated schematically.

The blood circuit 2 further comprises a regulation assembly 9 that regulates, i.e., is configured to selectively allow or interrupt the flow of blood through each treatment seat 30 (consequently through each filter 3). In other words, the regulation assembly 9 is associated with a respective treatment seat 30 (and consequently with a respective filter 3) and is configured to selectively activate or deactivate the passage of the blood through each seat 30, that is through the respective filter 3.

Downstream of the blood pump 7, the delivery duct 6 branches at a bifurcation 10 into a plurality of delivery branches 8, each of which connects an inlet (not illustrated) of the treatment seat 30 with the delivery of the blood pump 7. According to the example illustrated in Fig. 1, the filter 3 is installed in the respective seat 30 and the inlet of the treatment seat 30 is at the inlet mouth 4 of a respective filter 3. In particular, the inlet of the seat 30 allows connection between the outlet mouth 5 and the outlet branch 11.

According to the example illustrated, the delivery branches 8 are two and are indicated hereinafter with 81 and 811.

The blood circuit 2 further comprises an outlet branch 11 for each filter 3, to send treated blood towards the outlet U. According to the example illustrated, the outlet branches 11 are two, identified hereinafter with 11I and 11II. The outlet branches 11I and 11II reconnect in an outlet duct 12 that is connected with the outlet U.

According to the example illustrated in Fig. 1, the filter 3 is installed in the respective seat 30 and the outlet (not illustrated) of the treatment seat 30 is at the outlet mouth 5 of a respective filter 3. In particular, the outlet of the seat 30 allows connection between the outlet mouth 5 and the outlet branch 11.

Advantageously, the delivery branch 8 and the outlet branch 11 associated with a same seat 30, i.e., with a same filter 3, are at least partially side by side with one another.

According to the example illustrated in Fig. 1, the blood circuit 2 has two pairs of branches 131 and 1311. Each pair of branches 13 comprises a delivery branch 8 and the respective outlet branch 11 side by side with one another.

The blood circuit 2 of the type described above is produced, in a known manner, through flexible tubes made of biocompatible material.

Advantageously, the regulation assembly 9 comprises, for each pair of branches 13, a regulation device 14 configured to selectively and simultaneously adjust the flow of the blood through the respective delivery branch 8 and the respective outlet branch 11. For example, the regulation device 14 is configured to squeeze the tubes of the respective delivery branch 8 and of the respective outlet branch 11 from the outside. Advantageously, each regulation device 14 can be operated remotely, for example is electro-actuated.

According to what is illustrated in Fig. 1, the regulation assembly 9 comprises a support 15, which is suitable to house the tubes of each pair of branches 13 in predefined positions. The support 15 further comprises a housing for each regulation device 14. In particular, two regulation devices 14 are installed on the support 15, so that, in use, each regulation device 14 can selectively squeeze or release the tubes of a respective pair of branches 13.

The regulation assembly 9 further comprises an analysis unit 16 installed along the outlet duct 12 and configured to detect the presence, or absence, of given haematic components in the blood treated.

The blood circuit 2 further comprises a venous blood tray 17 arranged along the outlet duct 12 and interposed between the analysis unit 16 and the outlet U. The analysis unit 16 can comprise one or more sensors selected within a group of presence sensors that differ from one another for the type of component they detect. For example, the analysis unit 16 can comprise a sensor suitable to detect one or more of the following factors: pO2, pCo2, Urea, HCT, SatO2, pressure, pH. Naturally, the analysis unit 16 can comprise other types of sensors, such as temperature or density sensors. Preferable, the analysis unit 16 comprises a haematocrit detection sensor, hereinafter sensor HCT 27, which is configured to determine the concentration, or the percentage, of haematocrit in the blood. Moreover, the analysis unit 16 comprises a temperature sensor 28 and a haemoglobin sensor 29. Advantageously, the HCT sensor 27 is a multifunction sensor capable of detecting the temperature and concentration of haemoglobin, as well as the percentage of haematocrit.

The filtering system 1 further comprises a washing assembly 18, which in turn comprises a saline solution pre-dilution branch having a saline solution inlet IF1, connected in a known manner, not illustrated, to a saline solution delivery source (not illustrated). The pre-dilution branch 191 is configured to input the saline solution upstream of the filters 3, in particular upstream of the bifurcation 10. Moreover, the washing assembly 18 comprises a solution pump 201, in particular a peristaltic pump, arranged along the pre-dilution branch 191.

It must be noted that in the present description the terms upstream and downstream are utilised with reference to the direction of the blood flow through the blood circuit 2.

The washing assembly 18 further comprises a post-dilution branch 1911 having a saline solution inlet IF2, connected in a known manner, not illustrated, to a saline solution delivery source (not illustrated). The post-dilution branch 1911 inputs the saline solution downstream of the filters 3, in particular upstream of the venous blood tray 17.

The washing assembly 18 comprises a further solution pump 2011, in particular a peristaltic pump, arranged along the post-dilution branch 1911.

The washing assembly 18 further comprises an ultrafiltration branch 19III, which connects the outlet of a filter 3 with the outside. The ultrafiltration branch 19III is of known type and is illustrated schematically.

Advantageously, the filtering system 1 is configured so that the blood passes through the filter 3 in the same direction of flow as the ultrafiltration branch 19III. In this way no air is able to remain trapped inside the filter 3.

The regulation assembly 9 further comprises a control unit 21 that is configured to exchange data with: the blood pump 7, the bifurcation 10, each regulation device 141 and 1411, the analysis unit 16, and each solution pump 201 and 2011.

The regulation assembly 9 further comprises a plurality of input pressure detection elements 25. In particular, the regulation assembly 9 comprises a pressure detection element of the blood input to each filter 3; these detection elements are indicated in the figures with 251 and 2511, for the filter 31 and 311 respectively.

The regulation assembly 9 further comprises a plurality of output pressure detection elements 26. In particular, the regulation assembly 9 comprises a pressure detection element of the blood output from each filter 3; these detection elements are indicated in the figures with 261 and 2611, for the filter 31 and 311 respectively. For example, the pressure detection elements 25 and 26 are gauges of known type and illustrated schematically. Each pressure detection element 25 and 26 exchanges data with the control unit 21.

The control unit 21 comprises, in a known manner, a memory unit 22, a calculation unit 23 and a management unit 24, which is configured to exchange data externally, for example with the operator.

The management unit 24 can be produced in a plurality of different ways. For example, the management unit 24 can comprise a user interface, for example a display, to exchange input and output data with an operator. The management unit 24 can comprise automatic data reading means, such as optical readers or RFID systems.

Each operator, through the interface or the automatic reading means, can set one or more of the processing and operating parameters listed above. For example, each operator can set personalised data for each specific patient, or each operator can set data for each specific treatment.

Preferable, the processing parameters comprise:
- type of treatment to be performed;
- type of blood circuit 2;
- threshold for activating filter 3 change;
- threshold for interrupting filter 3 washing.
While the operating parameters comprise:
- velocity of each blood pump 7;
- velocity of each solution pump 20;
- filter 3 change;
- type of filter 3 to pass through;
- pressure of the blood input into each filter 3;
- pressure of the blood output from each filter 3;
- activation of filter 3 washing;
- type of filter 3 to be washed.

Moreover, the analysis unit 16 is configured to detect continuously, i.e., instant by instant, the percentage of haematocrit in the patient's blood. In particular, due to the haematocrit data detected by the haematocrit sensor HCT 27, the analysis unit 16 is able to provide a particularly accurate response in relation to the percentage of haematocrit, which in the filter change procedure enables precise measurement of the amount of saline solution introduced in the blood circuit. Advantageously, the analysis unit is able to detect percentages of haematocrit that range from 5% to over 60%.

The memory unit 22 is configured to contain association data between treatment parameters to be performed and operating parameters. The calculation unit 23 exchanges data with the memory unit 22 and the management unit 24 and determines operating and/or treatment parameters through said association data. Moreover, the memory unit 22 is able to store data, in particular data relating to the percentage of haematocrit detected by the analysis unit 16. Advantageously, the calculation unit 23 is configured to determine the variation of the percentage of haematocrit during treatment.

Operation of the filtering system 1 is described below.

In use, an operator enters the treatment and/or operating parameters, related to the given patient or to the given treatment to be performed, through said management unit. The parameters can be entered in a known manner, manually or automatically through specific readers.

Moreover, both the filters 31 and 311 are installed in a known manner in the filtering system 1.

The filters 31 and 311 are then subjected to a washing step.

During the washing step, the saline solution is input into the blood circuit 2 is conveyed first through a filter 31 and, subsequently, activating the regulation devices 14, through the other filter 311.

Then, saline solution is input by means of the solution pump 201 into the blood circuit 2 upstream of the bifurcation 10. Also in this case, washing of the filters 31 and 311 takes place alternately, activating the regulation devices 14.

The washing step is performed according to the filter 3 manufacturers' specifications.

The washing step is followed by a treatment step.

During the treatment step, a regulation device 141 (or 1411) is adjusted so as to allow the passage of blood through the respective pair of branches 131 (or 1311). Simultaneously, the other regulation device 14II (or 141) is adjusted so as to interrupt the passage of blood through the respective pair of branches 1311 (or 131). The term interrupt substantially means squeezing the respective pair of branches 13 externally to prevent the flow of blood.

Therefore, during the treatment step the blood is conveyed to a filter 31 (or 311), while the other filter 311 (or 31) is isolated.

During the treatment step the analysis unit 16 determines the percentage of basal haematocrit of the patient. The control unit 21 reprocesses the treatment and/or operating parameters based on the percentage values of basal haematocrit. Advantageously, the data detected by the analysis unit 16 allow the basal haematocrit value of the patient to be set in real time and, consequently, allow the treatment and/or operating parameters to be automatically adapted instantaneously through the control unit 21. In this way, the efficiency of the treatment is optimised and any setting errors of the filtering system 1 are eliminated, as will be better illustrated below.

The analysis unit 16 constantly determines during the treatment step the percentage of haematocrit in the blood. In particular, the percentage of haematocrit is continually reduced, during the return step of the blood. Preferably, the decrease in the percentage of haematocrit is linear in time. In other words, the reduction in the percentage of haematocrit should have a linear trend decreasing in time with a constant slope.

Advantageously, variations of the slope with respect to an ideal value detect potential malfunctions of the filter change system. For example, if the slope is different with respect to an ideal value, this can either mean that the dilution is not taking place or that it is excessive. Therefore, advantageously, the slope of the variation of the haematocrit value is an instrument for checking both the quantitative and qualitative trend of the filter change procedure.

Advantageously, the automatic filter change is performed when the analysis unit 6 detects a percentage of haematocrit below a preset threshold value. For example, the automatic filter change is performed when the percentage of haematocrit of the blood decreases, by a predefined value, with respect to the percentage of basal haematocrit of the patient detected during the washing step.

When the automatic filter change step is activated by the control unit 21, the sub-step of returning the blood present in the filter 31 (or 311) to the patient and the substep of switching the regulation devices 141 and 1411 are carried out.

The automatic filter change step can be activated as a function of the using time of each filter 3. In other words, the automatic filter change step can be activated after a predefined operating period of each filter 3. Alternatively, the automatic filter change step can be activated as a function of the pressure drop determined between the inlet and the outlet of each filter. In other words, the automatic filter change step can be adjusted as a function of the value of the difference between the pressures detected by the input 25 and output 26 pressure detection elements of each filter 3.

To perform the substep of returning the blood to the patient, the blood pump 7 is stopped. The saline solution is then delivered through the pre-dilution branch 191 upstream of the bifurication 10.

Delivery of the saline solution through the pre-dilution branch 191 continues until the parameters detected by the analysis unit 16 satisfy predefined requirements. For example, the return substep continues until the value of the blood detected by the analysis unit 16 with HCT or another relevant parameter is not below a predefined value.

The return sub-step is followed by the switching sub-step. During the switching sub-step, the flow of saline solution through the pre-dilution branch 191 is interrupted. During the switching sub-step each regulation device 14 is switched so as to reverse its operation. Therefore, with reference to the above, the regulation device 1411 (or 141) is activated to allow the blood to flow through the respective pair of branches 1311 (or 131), while the other regulation device 141 (or 1411) is activated to interrupt the flow of blood through the respective pair of branches 131 (or 1311) . Therefore, due to this adjustment by the regulation devices 14, the blood is conveyed to the filter 311 (or 31) that was isolated in the previous step, while the other initially operating filter 31 (or 311) is isolated. Following the switching sub-step, a further treatment step can be performed, during which the blood is delivered to the other filter 311 (or 3I).

Advantageously, the filtering system 1 of the type described above enables the treatment of the patient's blood to be performed during two subsequent treatment steps, each treatment step utilising a respective filter 3. Advantageously, the two treatment steps of the above type are performed directly one after the other without the need to carry out common priming activities.

Alternatively to the above, the filtering system 1 can have only one filter 3 during the initial treatment step, while a by-pass element is installed in the seat 30 of the other filter 3. In this case, during the switching sub-step the by-pass element is removed and replaced with a filter 3.

In this case, the automatic filter change step comprises the following sequence of sub-steps: replacement of the bypass element with a new filter; washing of the new filter; elimination of the washing solution by filtration through the membrane; return; and switching.

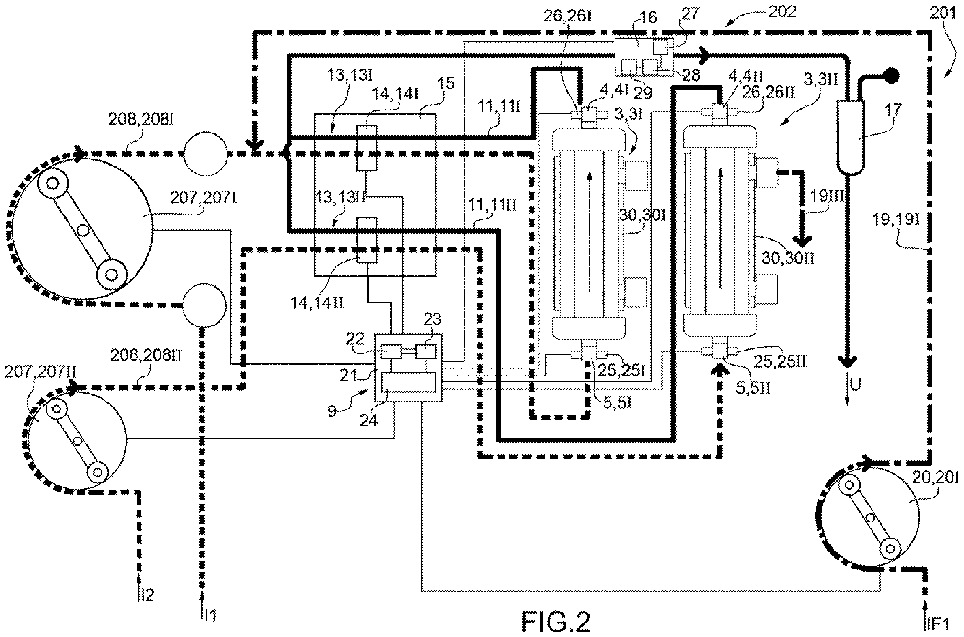

In Fig. 2, the numeral 201 indicates a further filtering system.

In Fig. 2, the components shared with the filtering system 1 of the type described above have the same numbers and are considered comprised in the filtering system 201, without listing them again.

The filtering system 201 comprises two blood pumps 2071 and 207II, while the blood circuit 202 comprises two delivery branches 2081 and 208II, each of which is connected in a known manner, not illustrated, to a respective inlet I1 and 12. In this way, each filter 31 and 311 is supplied directly by a respective source and two filtering processes can be carried out simultaneously.

In addition to the above description relating to the possibility of replacing the filters 3 during use of the filtering system 1, the filtering system 201 also allows treatment of the blood in parallel. This can be advantageous, for example, in the case in which an increase in the efficiency of the change is required, or in which two different treatments must be performed, such as haemofiltration and haemoperfusion, in which filters with different characteristics of duration or different blood flows are used.

Advantageously, in the filtering system 201 each filter 31, 311 can be emptied, returning the blood to the patient autonomously from the other. In this case, each filter 31, 311 is emptied by exploiting the corresponding blood pump 2071 and 207II, respectively.

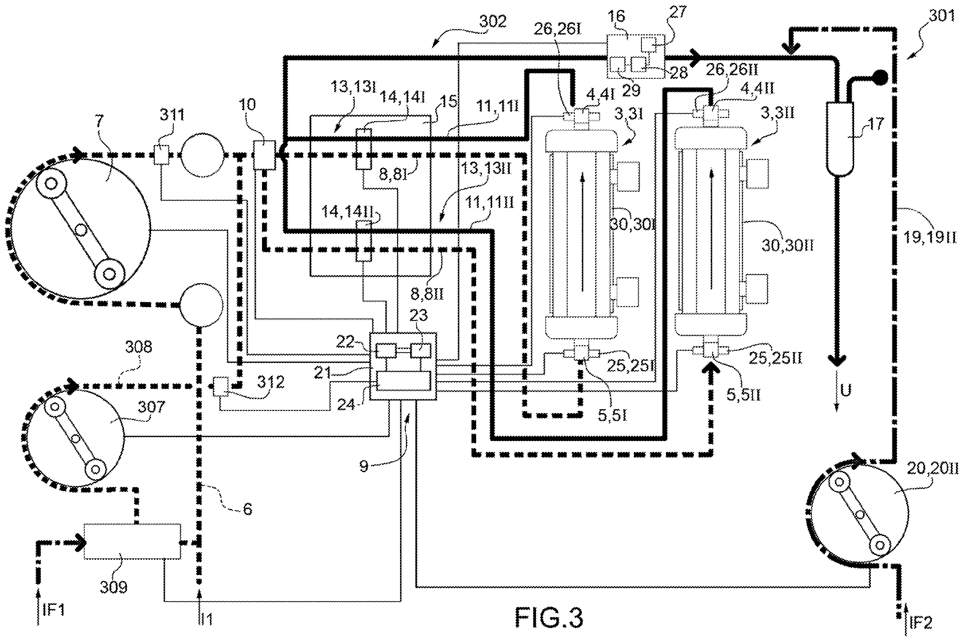

In Fig. 3, the numeral 301 indicates an embodiment of a filtering system according to the present invention. In Fig. 3, the components shared with the filtering system 1 of the type described above have the same numbers and are considered comprised in the filtering system 301 without listing them again.

The filtering system 301 comprises, in particular, an auxiliary pump 307 that can operate, according to needs, as blood pump or as solution pump.

The blood circuit 302 further comprises an auxiliary branch 308 that flows into the delivery duct 6 upstream of the bifurcation 10. The fluid that passes through the auxiliary branch 308 is pushed by the auxiliary pump 307.

The blood circuit 302 has, as indicated above, a saline solution inlet IF1 and an inlet I for blood and comprises a valve element 309 that selectively connects, according to need, the auxiliary branch 308 with the saline solution inlet IF1 or with the inlet I1 for blood.

In addition to the description above relating to the possibility of replacing the filters 3 during use of the filtering system 1, during use in the filtering system 301 the auxiliary pump 307 and the auxiliary branch 308 can be utilised either to increase the blood flow to 100% (if the blood pump 6 and the auxiliary pump 307 are the same) or to deliver, in conformity with the filtering system 1, the saline solution upstream of the filters 3.

Use of the auxiliary pump 307 and of the auxiliary branch 308 to deliver blood can be particularly advantageous in treatments to remove CO2 combined with a CRRT, or not, where a high blood flow is important to increase the efficiency of CO2 removal.

Advantageously, the auxiliary pump 307 can be phased with respect to the blood pump 7. In this way, it is possible to optimise the constancy of the blood flow and prevent pressure peaks. Due to the presence of the blood pump 7 and of the auxiliary pump 307 in parallel, it is possible to reduce stress on the blood and to reduce haemolysis.

Preferably, the filtering system 301 comprises pressure sensors 311 and 312 arranged along the delivery duct 6 downstream of the blood pump 7 and, respectively, along the delivery branch 308 downstream of the auxiliary pump 307. The pressure sensors 311 and 312 exchange data with the control unit 21, which is configured to determine the conditions of use and prevent any breakages of the blood circuit 302.

Advantageously, the presence of the auxiliary pump 307 allows a reduction in the stresses in the blood circuit 302; in fact, by offsetting the rollers of the auxiliary pump 307 by 90° with respect to the rollers of the blood pump 7 it is possible to reduce the pressure oscillations in the blood circuit 302.

Advantageously, the presence of the analysis unit 16 comprising the HCT sensor 27 along the outlet duct 12 allows the percentage of haematocrit to be detected, instant by instant; this measurement helps: to automatically determine the value of basal haematocrit of the patient and consequently to determine the operating parameters; to determine when the filter change must be activated; to determine whether washing of the filter 1 has terminated. Therefore, the filtering system 1 comprising the HCT sensor 27 allows automation of the filter change system and ensures the correctness and safety of the process.

## Claims

1. A filtering system for continuous renal replacement therapies having an inlet (I; I1) for the fluid to be treated, namely blood, and an outlet (U) for the treated fluid; the filtering system (301) comprising a blood circuit (302), which in turn comprises, a plurality of treatment seats (30; 301, 3011), which are interposed between said inlet (I; I1) and said outlet (U); each treatment seat (30) being connected to said inlet (I; I1, 12) through a respective delivery branch (8;); each treatment seat (30) being connected to said outlet (U) through a respective outlet branch (11); said seats (30; 301, 3011) being connected to said outlet (U) in parallel to each other; the filtering system (301) comprising a regulation assembly (9), which selectively regulates the fluid flow through each treatment seat (30; 301, 3011); wherein the treatment seats (30; 301, 3011) are connected to a delivery duct (6) and an outlet duct (12) in parallel to each another; wherein the blood circuit (302) comprises a pump (7), which is configured to push the fluid along said delivery duct towards all said treatment seats (30; 30I, 30II); **characterized in that** the blood circuit (302) comprises an auxiliary branch (308), which can be selectively connected either to an inlet (II) for the fluid to be treated or to a saline solution inlet (IF1); wherein said auxiliary branch (308) is connected to said delivery duct (6) upstream of said treatment seats (30; 301, 3011); wherein the blood circuit (302) comprises an auxiliary pump (307), which is configured to push the fluid to be treated or the saline solution towards said delivery duct (6); wherein said auxiliary pump (307) exchanges data with said control unit (21).

2. A filtering system according to claim 1, wherein the regulation assembly (9) comprises a regulation device (14; 141, 1411) for each seat (30; 301, 3011); wherein each regulation device (14; 141, 1411) regulates, namely interrupts or enables, the fluid flow through the respective seat (30; 301, 3011); wherein the regulation assembly (9) comprises a control unit (21) and each regulation device (14; 141, 1411) exchanges data with said control unit (21).

3. A filtering system according to claim 2, wherein the delivery branches (8, 8I, 8II) and the outlet branches (U) are flexible tubes; wherein the delivery branch (8, 8I, 8II) and the outlet branch (11; 11I, 11II) associated with a same seat (30; 30I, 30II) are at least partially side by side one another; wherein each regulation device (14; 14I, 14II) is configured to squeeze or release simultaneously the respective delivery branch (8I; 8II) and the respective outlet branch (11I; 11II).

4. A filtering system according to claim 2, wherein the regulation device (9) comprises, furthermore, an analysis unit (16), which is installed along said blood circuit (302) and downstream, in respect to the fluid flowing direction, of said treatment seats (30; 30I, 30II); wherein the analysis unit (16) detects predefined parameters of the fluid; wherein said control unit (21) exchanges data with said analysis unit (16) and each regulation device (14; 14I, 14II); wherein said control unit (21) operates each regulation device (14; 14I, 14II) as a function of the parameters detected by said analysis unit (16).

5. A filtering system according to claim 4, wherein the analysis unit (16) comprises a haematocrit sensor (27) that is configured to detect the percentage of haematocrit in the fluid; wherein said control unit (21) is configured to determine the basal haematocrit value as a function of the data exchanged with said haematocrit detection sensor (27) during a treatment step.

6. A filtering system according to claim 5, wherein said control unit (21) is configured to operate each regulation device (14; 14I, 14II) as a function of the data detected by the haematocrit sensor (27).

7. A filtering system according to claim 5 or 6, wherein said control unit (21) is configured to determine any malfunctions in the blood circuit (302) as a function of the data detected by the haematocrit sensor (27).

8. A filtering system according to one of claims 4 to 7, wherein the regulation assembly (9) comprises pressure detection means (25, 25I, 2511; 26, 26I, 26II), which detect, in use, the fluid pressure along the delivery branch (8, 8I, 8II) and the outlet branch (11; 11I, 11II) of each treatment seat (30; 30I, 30II); wherein said pressure detection means (25; 25I, 25II; 26; 26I, 26II) exchange data with the control unit (21); wherein said control unit (21) operates each regulation device (14; 14I, 14II) as a function of the parameters detected by said pressure detection means (25; 25I, 25II; 26; 26I, 26II).

9. A filtering system according to one of claims 4 to 8, wherein the blood circuit (302) comprises, furthermore, one or more peristaltic pumps (7), each configured to push said fluid along said blood circuit (302); wherein each pump (7) exchanges data with the control unit (21); wherein the control unit (21) regulates the operation, for example the rotation speed, of each pump (7).

10. A filtering system according to one of the claims from 2 to 9, wherein the control unit (21) comprises a memory unit (22), a calculation unit (23) and a managing unit (24), which exchanges data with an operator; wherein the memory unit (22) contains association data for the association between operating parameters and treatment parameters; wherein the calculation unit (23) exchanges data with the managing unit (24) and the memory unit (22).

11. A filtering system according to any of the preceding claims, wherein the blood circuit (302) comprises a valve element (309) to selectively connect the auxiliary branch (308) to the saline solution inlet (IF1) or to said inlet (I1).

12. A filtering system according to any of the preceding claims and comprising a first and a second pressure sensor (311; 312), each of which is arranged along said delivery duct (6) downstream of said pump (7) and, respectively, along said delivery branch (308) downstream of the auxiliary pump (307); wherein said first and said second pressure sensors (311; 312) exchange data with the control unit (21), which is configured to determine, as a function of the data exchanged with said pressure sensors (311, 312) any critical operating conditions of said blood circuit (302) .

## Patentansprüche

1. Filtersystem für kontinuierliche Nierenersatztherapien, das einen Einlass (I; I1) für das zu behandelnde Fluid, nämlich Blut, und einen Auslass (U) für das behandelte Fluid aufweist; wobei das Filtersystem (301) einen Blutkreislauf (302) umfasst, der wiederum eine Vielzahl von Behandlungssitzen (30; 30I, 30II) umfasst, die zwischen dem Einlass (I; I1) und dem Auslass (U) zwischengeschaltet sind; wobei jeder Behandlungssitz (30) mit dem Einlass (I; I1, I2) durch einen entsprechenden Lieferzweig (8;) verbunden ist; wobei jeder Behandlungssitz (30) mit dem Auslass (U) durch einen entsprechenden Auslasszweig (11) verbunden ist; wobei die Sitze (30; 30I, 30II) parallel zueinander mit dem Auslass (U) verbunden sind; wobei das Filtersystem (301) eine Regulierungsanordnung (9) umfasst, die die Fluidströmung durch jeden Behandlungssitz (30; 30I, 30II) selektiv regelt; wobei die Behandlungssitze (30; 30I, 30II) mit einem Lieferkanal (6) und einem Auslasskanal (12) parallel zueinander verbunden sind; wobei der Blutkreislauf (302) eine Pumpe (7) umfasst, die dazu ausgestaltet ist, das Fluid entlang des Lieferkanals hin zu sämtlichen Behandlungssitzen (30; 30I, 30II) zu drücken;
**dadurch gekennzeichnet, dass** der Blutkreislauf (302) einen Nebenzweig (308) umfasst, der selektiv mit entweder einem Einlass (I1) für das zu behandelnde Fluid oder mit einem Kochsalzlösungseinlass (IF1) verbunden werden kann; wobei der Nebenzweig (308) mit dem Lieferkanal (6) stromaufwärts der Behandlungssitze (30; 30I, 30II) verbunden ist; wobei der Blutkreislauf (302) eine Nebenpumpe (307) umfasst, die so ausgestaltet ist, dass sie das zu behandelnde Fluid oder die Salzlösung hin zu dem Lieferkanal (6) drückt; wobei die Nebenpumpe (307) Daten mit der Steuereinheit (21) austauscht.

2. Filtersystem nach Anspruch 1, wobei die Regulierungsanordnung (9) eine Regulierungsvorrichtung (14; 14I, 14II) für jeden Sitz (30; 30I, 30II) umfasst; wobei jede Regulierungsvorrichtung (14; 14I, 14II) die Fluidströmung durch den entsprechenden Sitz (30; 30I, 30II) reguliert, nämlich unterbricht oder ermöglicht; wobei die Regulierungsanordnung (9) eine Steuereinheit (21) umfasst und jede Regulierungsvorrichtung (14; 14I, 14II) Daten mit der Steuereinheit (21) austauscht.

3. Filtersystem nach Anspruch 2, wobei die Lieferzweige (8, 8I, 8II) und die Auslasszweige (U) Schläuche sind; wobei der Lieferzweig (8, 8I, 8II) und der Auslasszweig (1I; 11I, 11II), die einem selben Sitz (30; 30I, 30II) zugehörig sind, zumindest teilweise nebeneinanderliegen; wobei jede Regulierungsvorrichtung (14; 14I, 14II) so ausgestaltet ist, dass sie den entsprechenden Lieferzweig (8I; 8II) und den entsprechenden Auslasszweig (11I; 11II) gleichzeitig quetscht oder freigibt.

4. Filtersystem nach Anspruch 2, wobei die Regulierungsvorrichtung (9) ferner eine Analyseeinheit (16) umfasst, die entlang des Blutkreislaufs (302) und in Bezug auf die Fluidströmungsrichtung stromabwärts der Behandlungssitze (30; 30I, 30II) eingerichtet ist; wobei die Analyseeinheit (16) vorbestimmte Parameter des Fluids detektiert; wobei die Steuereinheit (21) Daten mit der Analyseeinheit (16) und jeder Regulierungsvorrichtung (14; 14I, 14II) austauscht; wobei die Steuereinheit (21) jede Regulierungsvorrichtung (14; 14I, 14II) als eine Funktion der von der Analyseeinheit (16) detektierten Parameter betreibt.

5. Filtersystem nach Anspruch 4, wobei die Analyseeinheit (16) einen Hämatokritsensor (27) umfasst, der so ausgestaltet ist, das er einen Prozentsatz von Hämatokrit in dem Fluid detektiert; wobei die Steuereinheit (21) so ausgestaltet ist, dass sie den Hämatokrit-Basalwert als eine Funktion der mit dem Hämatokrit-Detektionssensor (27) während eines Behandlungsschritts ausgewählten Daten bestimmt.

6. Filtersystem nach Anspruch 5, wobei die Steuereinheit (21) so ausgestaltet ist, dass sie jede Regulierungsvorrichtung (14; 14I, 14II) als eine Funktion der von dem Hämatokritsensor (27) detektierten Daten betreibt.

7. Filtersystem nach Anspruch 5 oder 6, wobei die Steuereinheit (21) so ausgestaltet ist, dass sie etwaige Störungen im Blutkreislauf (302) als eine Funktion der von dem Hämatokritsensor (27) detektierten Daten bestimmt.

8. Filtersystem nach einem der Ansprüche 4 bis 7, wobei die Regulierungsanordnung (9) Druckdetektionsmittel (25, 25I, 25II; 26, 26I, 26II) umfasst, die bei der Verwendung den Fluiddruck entlang des Lieferzweigs (8, 8I, 8II) und des Auslasszweigs (11; 11I, 11II) jedes Behandlungssitzes (30; 30I, 30II) detektieren; wobei die Druckdetektionsmittel (25; 25I, 25II; 26; 26I, 26II) Daten mit der Steuereinheit (21) austauschen; wobei die Steuereinheit (21) jede Regulierungsvorrichtung (14; 14I, 14II) als eine Funktion der von den Druckdetektionsmitteln (25; 25I, 25II; 26; 26I, 26II) detektierten Parameter betreibt.

9. Filtersystem nach einem der Ansprüche 4 bis 8, wobei der Blutkreislauf (302) ferner eine oder mehrere Peristaltikpumpen (7) umfasst, die jeweils so ausgestaltet sind, dass sie das Fluid entlang des Blutkreislaufs (302) drücken; wobei jede Pumpe (7) Daten mit der Steuereinheit (21) austauscht; wobei die Steuereinheit (21) den Betrieb, zum Beispiel die Drehzahl, jeder Pumpe (7) reguliert.

10. Filtersystem nach einem der Ansprüche 2 bis 9, wobei die Steuereinheit (21) eine Speichereinheit (22), eine Berechnungseinheit (23) und eine Verwaltungseinheit (24) umfasst, die Daten mit einem Bediener austauscht; wobei die Speichereinheit (22) Verknüpfungsdaten für die Verknüpfung zwischen Betriebsparametern und Behandlungsparametern enthält; wobei die Berechnungseinheit (23) Daten mit der Verwaltungseinheit (24) und der Speichereinheit (22) austauscht.

11. Filtersystem nach einem der vorhergehenden Ansprüche, wobei der Blutkreislauf (302) ein Ventilelement (309) zum selektiven Verbinden des Nebenzweigs (308) mit dem Salzlösungseinlass (IF1) oder dem Einlass (I1) umfasst.

12. Filtersystem nach einem der vorhergehenden Ansprüche und das einen ersten und einen zweiten Drucksensor (311; 312) umfasst, die jeweils entlang des Lieferkanals (6) stromabwärts der Pumpe (7) beziehungsweise entlang des Lieferzweigs (308) stromabwärts der Nebenpumpe (307) angeordnet sind; wobei der erste und der zweite Drucksensor (311; 312) Daten mit der Steuereinheit (21) austauschen, die so ausgestaltet ist, dass sie als eine Funktion der mit den Drucksensoren (311, 312) ausgetauschten Daten beliebige kritische Betriebsbedingungen des Blutkreislaufs (302) bestimmt.

## Revendications

1. Système de filtration pour des thérapies de remplacement rénal en continu, présentant une entrée (I ; I1) pour le fluide à traiter, à savoir le sang, et une sortie (U) pour le fluide traité ; le système de filtration (301) comprenant un circuit sanguin (302), lequel comprend à son tour une pluralité de sièges de traitement (30 ; 30I, 30I1), qui sont interposés entre ladite entrée (I ; I1) et ladite sortie (U) ; chaque siège de traitement (30) étant relié à ladite entrée (I ; I1, I2) par le biais d'une branche de distribution respective (8) ; chaque siège de traitement (30) étant relié à ladite sortie (U) par une branche de sortie respective (11) ; lesdits sièges de traitement (30 ; 30I, 30II) étant reliés à ladite sortie (U) en parallèle les uns avec les autres ; le système de filtration (301) comprenant un ensemble de régulation (9), qui régule sélectivement l'écoulement de fluide à travers chaque siège de traitement (30 ; 30I, 30II) ; dans lequel les sièges de traitement (30 ; 30I, 30II) sont reliés à un conduit de distribution (6) et à un conduit de sortie (12) en parallèle l'un avec l'autre ; dans lequel le circuit sanguin (302) comprend une pompe (7), qui est configurée de manière à pousser le fluide le long dudit conduit de distribution vers la totalité desdits sièges de traitement (30 ; 30I, 30II) ; **caractérisé en ce que** le circuit sanguin (302) comprend une branche auxiliaire (308), qui peut être reliée sélectivement soit à une entrée (I1) pour le fluide à traiter, soit à une entrée de solution saline (IF1) ; dans lequel ladite branche auxiliaire (308) est reliée audit conduit de distribution (6) en amont desdits sièges de traitement (30 ; 30I, 30II) ; dans lequel le circuit sanguin (302) comprend une pompe auxiliaire (307), qui est configurée de manière à pousser le fluide à traiter ou la solution saline vers ledit conduit de distribution (6) ; dans lequel ladite pompe auxiliaire (307) échange des données avec une unité de commande (21).

2. Système de filtration selon la revendication 1, dans lequel l'ensemble de régulation (9) comprend un dispositif de régulation (14 ; 14I, 14II) pour chaque siège (30 ; 30I, 30II) ; dans lequel chaque dispositif de régulation (14 ; 14I, 14II) régule, à savoir interrompt ou active, l'écoulement de fluide à travers le siège respectif (30 ; 30I, 3011) ; dans lequel l'ensemble de régulation (9) comprend une unité de commande (21) et chaque dispositif de régulation (14 ; 14I, 14II) échange des données avec ladite unité de commande (21).

3. Système de filtration selon la revendication 2, dans lequel les branches de distribution (8, 8I, 8II) et les branches de sortie (U) sont des tubes flexibles ; dans lequel la branche de distribution (8, 8I, 8II) et la branche de sortie (11 ; 11I, 11II) associées à un même siège (30 ; 30I, 30II) sont au moins partiellement côte à côte l'une et l'autre ; dans lequel chaque dispositif de régulation (14 ; 14I, 14II) est configuré de manière à comprimer ou libérer simultanément la branche de distribution respective (8I; 8II) et la branche de sortie respective (11I ; 11II).

4. Système de filtration selon la revendication 2, dans lequel le dispositif de régulation (9) comprend, en outre, une unité d'analyse (16), qui est installée le long dudit circuit sanguin (302) et en aval, par rapport au sens d'écoulement du fluide, desdits sièges de traitement (30 ; 30I, 30II) ; dans lequel l'unité d'analyse (16) détecte des paramètres prédéfinis du fluide ; dans lequel ladite unité de commande (21) échange des données avec ladite unité d'analyse (16) et chaque dispositif de régulation (14 ; 14I, 14II) ; dans lequel ladite unité de commande (21) fait fonctionner chaque dispositif de régulation (14 ; 14I, 14II) en fonction des paramètres détectés par ladite unité d'analyse (16).

5. Système de filtration selon la revendication 4, dans lequel l'unité d'analyse (16) comprend un capteur d'hématocrite (27) qui est configuré de manière à détecter le pourcentage d'hématocrite dans le fluide ; dans lequel ladite unité de commande (21) est configurée de manière à déterminer la valeur d'hématocrite basale en fonction des données échangées avec ledit capteur d'hématocrite (27) pendant une étape de traitement.

6. Système de filtration selon la revendication 5, dans lequel ladite unité de commande (21) est configurée de manière à faire fonctionner chaque dispositif de régulation (14 ; 14I, 14II) en fonction des données détectées par le capteur d'hématocrite (27).

7. Système de filtration selon la revendication 5 ou 6, dans lequel ladite unité de commande (21) est configurée de manière à déterminer des dysfonctionnements quelconques dans le circuit sanguin (302) en fonction des données détectées par le capteur d'hématocrite (27).

8. Système de filtration selon l'une quelconque des revendications 4 à 7, dans lequel l'ensemble de régulation (9) comprend des moyens de détection de pression (25, 25I, 25II ; 26, 26I, 26II), qui détectent, en cours d'utilisation, la pression fluidique le long de la branche de distribution (8, 8I, 8II) et de la branche de sortie (11 ; 11I, 11II) de chaque siège de traitement (30 ; 30I, 3011) ; dans lequel lesdits moyens de détection de pression (25, 25I, 25II ; 26, 26I, 26II) échangent des données avec l'unité de commande (21) ; dans lequel ladite unité de commande (21) fait fonctionner chaque dispositif de régulation (14 ; 14I, 14II) en fonction des paramètres détectés par lesdits moyens de détection de pression (25, 25I, 25II ; 26, 26I, 2611).

9. Système de filtration selon l'une quelconque des revendications 4 à 8, dans lequel le circuit sanguin (302) comprend, en outre, une ou plusieurs pompes péristaltiques (7), chacune configurée de manière à pousser ledit fluide le long dudit circuit sanguin (302) ; dans lequel chaque pompe (7) échange des données avec l'unité de commande (21) ; dans lequel l'unité de commande (21) régule le fonctionnement, par exemple la vitesse de rotation, de chaque pompe (7).

10. Système de filtration selon l'une quelconque des revendications 2 à 9, dans lequel l'unité de commande (21) comprend une unité de mémoire (22), une unité de calcul (23) et une unité de gestion (24), qui échange des données avec un opérateur ; dans lequel l'unité de mémoire (22) contient des données d'association pour l'association entre des paramètres de fonctionnement et des paramètres de traitement ; dans lequel l'unité de calcul (23) échange des données avec l'unité de gestion (24) et l'unité de mémoire (22).

11. Système de filtration selon l'une quelconque des revendications précédentes, dans lequel le circuit sanguin (302) comprend un élément de valve (309) pour relier sélectivement la branche auxiliaire (308) à l'entrée de solution saline (IF1) ou à ladite entrée (I1).

12. Système de filtration selon l'une quelconque des revendications précédentes, comprenant un premier capteur de pression et un second capteur de pression (311 ; 312), lesquels sont chacun agencés le long dudit conduit de distribution (6) en aval de ladite pompe (7) et, respectivement, le long de ladite branche de distribution (308) en aval de la pompe auxiliaire (307) ; dans lequel lesdits premier et second capteurs de pression (311 ; 312) échangent des données avec l'unité de commande (21), qui est configurée de manière à déterminer, en fonction des données échangées avec lesdits capteurs de pression (311, 312), toute condition de fonctionnement critique dudit circuit sanguin (302).
